# EUROPÄISCHE PATENTSCHRIFT

(11) **EP 3 172 439 B1**
(45) Veröffentlichungstag und Bekanntmachung des Hinweises auf die Patenterteilung: **11.07.2018**
(21) Anmeldenummer: 15738395.1
(22) Anmeldetag: 22.07.2015
(51) Int. Cl.: F04B 41/06, A61M 1/00, F04F 5/00

(54) **SAUGVORRICHTUNG FÜR MEDIZINISCHE UND INDUSTRIELLE ZWECKE**
SUCTION DEVICE FOR MEDICAL AND INDUSTRIAL PURPOSES
DISPOSITIF D'ASPIRATION À DES FINS MÉDICALES ET INDUSTRIELLES

(30) Priorität: 25.07.2014 EP 14178649
(43) Veröffentlichungstag der Anmeldung: 31.05.2017
(73) Patentinhaber: BPR Swiss GmbH, 3672 Oberdiessbach (CH)
(72) Erfinder: MAURER, Marcel, CH-2560 Nidau (CH); MAURER, Marc, CH-3073 Gümligen (CH); RUEGSEGGER, Hans-Peter, CH-3673 Linden (CH)
(74) Vertreter: Rutz & Partner
(86) Internationale Anmeldenummer: PCT/EP2015/066745
(87) Internationale Veröffentlichungsnummer: WO 2016/012494

(56) Entgegenhaltungen:
- EP-A2- 1 310 267
- WO-A1-2010/054142
- DE-A1- 4 210 539
- US-A1- 2005 095 142

## Beschreibung

Die Erfindung betrifft eine Saugvorrichtung für medizinische, insbesondere dental-medizinische, und industrielle Zwecke.

Saugvorrichtungen werden in zahlreichen Bereichen der Industrie und der Medizin eingesetzt. Im Bereich der Dentalmedizin dienen Saugvorrichtungen der Erzeugung eines Unterdrucks an einer Absaugkanüle oder Saugleitung, durch die Flüssigkeit und Feststoffpartikel, insbesondere Amalgampartikel, aus dem Mund eines Patienten abgesaugt werden.

Wird von Seiten der Anwender ein leichtes, kleines und möglichst geräuscharmes Absaugsystem gefordert, werden in der Dental- und Medizintechnik bisher gasfördernde Verdrängerpumpen, wie Kolbenpumpen oder Membranpumpen verwendet. Soll die Saugvorrichtung in ein kleineres Gerät integriert werden, wird oft eine Strahlpumpe verwendet, wie dies in der US20090061384A1 beschrieben ist.

Aus der WO2010054142A1 ist eine Vorrichtung für ophtalmologische Zwecke, insbesondere zur Durchführung von Kataraktoperationen, bekannt, bei denen Teile einer zertrümmerten Linse durch eine Absaugleitung aus dem Auge entfernt werden. Dazu sind zwei Pumpen, eine peristaltische Pumpe und eine Venturipumpe sowie eine damit verbundene Steuervorrichtung vorgesehen. Mittels der Steuervorrichtung wird die jeweils geeignete Pumpe an die Absaugleitung angeschaltet, um die freigelegten Teile der Linse möglichst rasch abtransportieren zu können. Die peristaltische Pumpe dient dabei dem Abtransport grösserer Partikel, die mittels der Venturipumpe nicht erfasst werden können. Peristaltische Pumpen oder Schlauchquetschpumpen werden primär in DialyseGeräten und Herz-Lungen-Maschinen verwendet, sind aber für die Anwendung in der Dentaltechnik nicht geeignet. Die beiden Pumpen erzeugen während der Umschaltphase einen weiteren variablen Druck. Die Anordnung besitzt jedoch keine Vakuumkammer. Bisher eingesetzte Saugvorrichtungen, wie die genannten peristaltischen Pumpen benötigen am Arbeitspunkt, an dem das Absaugmaterial angesogen wird, üblicherweise einen Widerstand, um das für diese Geräte maximal mögliche Vakuum aufbauen zu können. Dies führt insbesondere bei medizinischen Anwendungen zu verschiedenen Problemen. Die Absaugkanüle muss sich zuerst einige Zeit vollständig in der abzusaugenden Flüssigkeit befinden bevor die Saugvorrichtung ihre volle Wirkung entfalten kann, weshalb bei einem medizinischen Eingriff unerwünschte Verzögerungen eintreten. Der Anwender muss gezielt nach einer Flüssigkeitsansammlung suchen, welche die Öffnung der Kanüle voll abdeckt. Die letzten Tropfen der abgesogenen Flüssigkeit fliessen zudem wieder zurück, da das Vakuum nicht mehr aufrechterhalten werden kann.
Um diese Nachteile zu vermeiden, wird oft eine Gebläsepumpe verwendet, die Luft durch drehende Schaufeln in einen Kanal transportiert. Gebläsepumpen erreichen einen hohen Volumenstrom jedoch nur ein geringes Vakuum und kann im Vergleich zu Ejektoren und Verdrängerpumpen weniger Flüssigkeit absaugen. Nachteilig bei Gebläsepumpen ist zudem, dass sowohl das Rad mit den drehenden Schaufeln als auch der zugehörige Antriebsmotor relativ gross und schwer sind. Ferner weisen Gebläsepumpen einen hohen Energiebedarf auf.
Der vorliegenden Erfindung liegt daher die Aufgabe zugrunde, eine verbesserte Saugvorrichtung für medizinische und industrielle Zwecke zu schaffen, bei der insbesondere die oben beschriebenen Nachteile bekannter Pumpvorrichtungen vermieden werden.

Die Saugvorrichtung soll es erlauben, Flüssigkeiten selbst dann verzögerungslos und vollständig absaugen zu können, wenn die Absaugkanüle nicht vollständig in die Flüssigkeit eingetaucht ist. Insbesondere sollen Flüssigkeitsansammlungen mittels der Absaugkanüle rasch und vollständig aufgenommen werden können, die tropfenförmig auf einer Oberfläche verteilt sind.

Die Saugvorrichtung soll ferner kompakt und einfach aufgebaut werden können, so dass sie nur ein geringes Gewicht aufweist und wenig Raum in Anspruch nimmt.

Weiterhin soll die Saugvorrichtung effizient mit geringem Energieverbrauch betrieben werden können.

Die Saugvorrichtung soll zudem universell einsetzbar sein und bestehende Systeme problemlos ersetzen können. Alternativ sollen bestehende Systeme einfach nachgerüstet werden können, um die erfindungsgemässen Vorteile zu erzielen.

Insbesondere soll die Saugvorrichtung in einem Behälter oder in einer Vakuumkammer einen hohen Unterdruck erzeugen können, sodass mittels einer daran angeschlossenen Absaugleitung Flüssigkeit, z.B. Flüssigkeit aus dem Mund eines Patienten während einer Dentalbehandlung, effizient abgesaugt und in den Behälter überführt werden kann.

Diese Aufgabe wird mit einer Saugvorrichtung gelöst, welche die in Anspruch 1 angegebenen Merkmale aufweist.

Vorteilhafte Ausgestaltungen der Erfindung sind in weiteren Ansprüchen angegeben.

Die insbesondere für medizinische und industrielle Anwendungen vorgesehene Saugvorrichtung, die dem Absaugen von Flüssigkeit und/oder Feststoffpartikeln dient, umfasst eine als Strahlpumpe, insbesondere als Venturi-Pumpe oder Ejektor-Pumpe, ausgebildeten ersten Saugeinheit, mittels der in einer ersten Anschlussleitung ein erster Unterdruck realisierbar ist.

Erfindungsgemäss **ist** eine als Verdrängerpumpe oder Gebläsepumpe ausgebildete zweite Saugeinheit vorgesehen, mittels der in einer zweiten Anschlussleitung ein zweiter Unterdruck realisierbar ist, wobei die erste und die zweite Anschlussleitung direkt oder über eine Vakuumkammer an eine Saugleitung angeschlossen sind, und wobei wenigstens eine Energieversorgungsvorrichtung mit der ersten und der zweiten Saugeinheit derart verbindbar oder verbunden ist, dass beide Saugeinheiten gemeinsam betrieben werden können, um gemeinsam einen dritten Unterdruck in der Saugleitung oder der Vakuumkammer zu erzeugen.

Die Prinzipien von Strahlpumpen, z.B. Venturi-Pumpen oder Ejektor-Pumpen, die als erste Saugeinheiten verwendbar sind, sind in den Wikipedia-Seiten http://de.wikipedia.org/wiki/Strahlpumpe und http://de.wikipedia.org/wiki/Venturi-Düse beschrieben. Bei beiden Strahlpumpen wird ein Treibmedium typischerweise durch eine Düse, eine Ejektor-Düse oder eine Venturi-Düse, geführt.

Verdrängerpumpen, die als zweite Saugeinheit verwendbar sind, sind in http://de.wikipedia.org/wiki/Pumpe beschrieben.

Es wurde festgestellt, dass durch die erfindungsgemässe Kopplung der ersten und der zweiten Saugeinheit, die auf unterschiedlichen physikalischen Prinzipien beruhen, ein dritter Unterdruck in der Saugleitung erzielt werden kann, mittels dessen Flüssigkeit, wie Blut oder Speichel, deutlich besser bei bekannten Saugvorrichtungen, insbesondere verzögerungsfrei und mit hohem Durchfluss abgesaugt werden kann. Bei Inbetriebnahme der Saugvorrichtung wird praktisch sofort die maximale Absaugleistung erzielt. Dabei ist es nicht notwendig, dass das abgesaugte Medium einen Strömungswiderstand bildet. Ebenso ist es nicht notwendig, dass die Saugleitung vollständig mit Flüssigkeit gefüllt ist. Flüssigkeit kann zudem bis auf den letzten Tropfen vollständig abgesaugt werden, wobei ein Rückfluss vermieden wird.

Durch die Verbindung von zwei Saugeinheiten, die nach unterschiedlichen Prinzipien arbeiten, können die Schwächen jeder Saugeinheit beseitigt werden. Durch die Kopplung der beiden Saugvorrichtungen resultiert somit nicht nur die erwartete Erhöhung, gegebenenfalls Verdopplung der Saugleistung, sondern eine weitere Verbesserung. Durch die Wechselwirkung der beiden Saugeinheiten wird das Saugverhalten jeder einzelnen Saugeinheit verbessert. Die Saugeinheiten unterstützen sich somit gegenseitig, so dass jede während des gemeinsamen Betriebs bessere Arbeitsergebnisse liefert. Durch den gemeinsamen Betrieb der Saugeinheiten verbessert sich ferner das Strömungsverhalten des abgesaugten Mediums oder Gas-Flüssigkeits-Gemischs über die gesamte Betriebsdauer bzw. Absaugdauer. Bereits mit Inbetriebnahme der Saugvorrichtung wird eine kräftige Strömung erzielt, weshalb die von beiden Saugeinheiten zur Verfügung gestellte Saugenergie wirkungsvoll auf das angesaugte Medium übertragen werden kann, um dieses effizient zu transportieren. Es werden deutlich höhere Durchflussraten und ein höherer Ansaugdruck erzielt.

Durch Anschluss der beiden Saugeinheiten an eine Vakuumkammer bzw. einen Unterdruckbehälter kann darin ein hoher Unterdruck erzeugt werden, sodass ein hohes Saugwirkung über eine an die Vakuumkammer angeschlossene Saugleitung auf eine Flüssigkeit z.B. im Mund eines Patienten einwirken kann. Flüssigkeit kann daher effizient abgesaugt und in die Vakuumkammer transferiert werden.

Besonders vorteilhaft ist die Kombination der ersten Saugeinheit mit einer als Verdrängerpumpe, z.B. als Membranpumpe oder Kolbenpumpe, ausgebildeten zweiten Saugeinheit. Bei dieser Kombination der Pumpsysteme erfolgt eine ideale Ergänzung zu einer Saugvorrichtung, die mit geringem Gewicht und geringen Abmessungen und zugleich hoher Saugleistung über die gesamte Betriebsdauer erzielt werden kann. Eine peristaltische Pumpe als zweite Saugeinheit ist hingegen nicht geeignet, da der erfindungsgemässe Erfolg nicht eintritt.

Durch die Kombination der beiden nach unterschiedlichen Prinzipien arbeitenden Saugeinheiten wird auch eine Erhöhung des Volumenstroms durch die erste Saugeinheit erzielt. Von der Verdrängerpumpe impulsweise zugeführtes Material gelangt verstärkt in den Volumenstrom der ersten Saugeinheit und wird von dieser erfasst und abtransportiert. Es resultiert somit eine vorteilhafte Wechselwirkungen der beiden Saugeinheiten.

Bei der Kombination der ersten Saugeinheit mit einer als Gebläsepumpe ausgebildeten zweiten Saugeinheit kann das Strömungsverhalten des transportierten Mediums vorteilhaft beeinflusst werden. Vorzugsweise wirkt die erste Saugeinheit in Zonen des von der Gebläsepumpe angetriebenen Medienstroms, in dem nicht-laminare Strömungen auftreten, z.B. an der Peripherie des rotierenden Förderwerkzeugs. Auf diese Weise wird durch die erste Saugeinheit nicht nur eine zusätzliche Saugleistung hinzugefügt, sondern die von der zweiten Saugeinheit bewirkte Medienströmung laminarisiert. Zusätzlich zum Strömungsanteil, welcher durch die erste Saugeinheit bewirkt wird, tritt ein zusätzlicher Strömungsanteil auf, welcher durch die Idealisierung der gesamten Medienströmung bewirkt wird.

Besonders vorteilhaft kann die erste Saugeinheit über ein Saugrohr auf die Medienströmung einwirken, das z.B. ringförmig an die Peripherie des rotierenden Werkzeugs bzw. Propellers anschliesst.

Die beiden Saugeinheiten sind vorzugsweise über zugehörige Anschlussleitungen und eine Kopplungseinheit an die Saugleitung angeschlossen. Die Kopplungseinheit teilt den angesaugten und aus dem Mediengemisch abgetrennten gasförmigen Medienstrom in zwei Teilströme auf, die den Saugeinheiten getrennt voneinander zugeführt werden. Durch Zusammenführung der Anschlussleitungen in der Kopplungseinheit oder einer Vakuumkammer bzw. Unterdruckkammer können die Saugleistungen der beiden Saugeinheiten vorteilhaft überlagert werden. Vorzugsweise liegt der Trennungspunkt der zwei Teilströme möglichst nahe bei der Saugleitung und vorzugsweise auch möglichst nahe bei der ersten Saugeinheit, wodurch eine optimale Wechselwirkung zwischen den Saugeinheiten erzielt wird. Das von den Teilströmen durchlaufene Raumvolumen zwischen dem Eingang zur Anschlussleitung einerseits und den Anschlussleitungen bzw. der Kopplungsstelle der Anschlussleitungen der beiden Saugeinheiten andererseits wird vorzugsweise möglichst klein gehalten, so dass Strömungsverluste vermieden werden.

Vorzugsweise wird zwischen der Saugleitung und den beiden Saugeinheiten eine aktive oder passive Medientrennvorrichtung vorgesehen, mittels der der Medienstrom in einen gasförmigen ersten Teilstrom und einen zweiten Teilstrom mit Flüssigkeit und Feststoffen aufgeteilt werden kann. Der zweite Teilstrom wird vorzugsweise weiter in Flüssigkeit und Feststoffe aufgeteilt.

Die Abtrennung der Flüssigkeit erfolgt vorzugsweise durch Nutzung kinetischer Energie und Schwerkraft, die auf die Flüssigkeit und die Feststoffe einwirken und diese aus dem Medienstrom auslösen. Aus der Saugleitung austretende Flüssigkeit wird in eine erste Richtung geschleudert und abgesondert, während der gasförmige Anteil des Medienstroms z.B. umgelenkt und zurückgeführt wird.

Dieser Vorgang des Wegschleuderns oder des Wegtransports von Flüssigkeit und Feststoffen kann auch mittels bewegter mechanischer Teile bewirkt werden, die in den Verlauf des Medienstroms eingefügt werden. Z.B. wird eine drehende Scheibe in den Medienstrom eingefügt, welche auftreffende Flüssigkeit und Feststoffe nach aussen schleudert und den verbleibenden gasförmigen Medienstrom nicht wesentlich behindert.

Vorzugsweise werden die Saugeinheiten einzeln oder über eine Kopplungsvorrichtung an wenigstens einen Anschlussport eines Aussenrohrs der Medientrennvorrichtung angeschlossen, wobei die Saugleitung oder ein daran vorgesehenes Verlängerungsstück das Aussenrohr vorzugsweise koaxial durchläuft und am Anschlussport vorbei geführt ist. Während der gasförmige Medienstrom aufgrund des von den Saugeinheiten bewirkten Unterdrucks praktisch widerstandslos zum Anschlussport zurückgeführt wird, gelingt es der Flüssigkeit und den Feststoffen nicht, zurück zum Anschlussport zu gelangen.

In vorzugsweisen Ausgestaltungen sind die Saugeinheiten einerseits und die Saugleitung andererseits an eine Vakuumkammer angeschlossen und darin miteinander gekoppelt. Die Vakuumkammer kann beliebig ausgestaltet und an die Anforderungen der Anwendung angepasst sein. Die Vakuumkammer kann ein sehr kleines Volumen aufweisen und durch ein Verbindungsstück der Rohrleitungen realisiert werden. Flüssigkeit und Feststoffe werden in diesem Fall durch eine Absaugpumpe aus der Vakuumkammer entfernt, in der die beiden Saugeinheiten einen Unterdruck erzeugen.

Die Vakuumkammer kann jedoch auch grösser ausgestaltet sein und den Anschluss weiterer Saugeinheiten, z.B. von mehreren seriell oder parallel angeordneten ersten Saugeinheiten erlauben und/oder der temporären Aufnahme von Flüssigkeit und/oder Feststoffen dienen, die vorzugsweise getrennt abgeführt, gegebenenfalls abgepumpt werden.

Insbesondere bei der Verwendung einer grösseren Vakuumkammer, die grössere Mengen an Flüssigkeit und Feststoffen aufnehmen kann, wird die erste Saugeinheit, die zweite Saugeinheit und/oder die Saugleitung vorzugsweise an eine Abdeckung angeschlossen, mittels der die Vakuumkammer abschliessbar ist. Auf diese Weise können alle wesentlichen Vorrichtungsteile zusammengefasst und in einfacher Weise montiert werden.

Im Bereitschaftszustand wird die Vakuumkammer vorzugsweise mittels eines Ventils abgeschlossen und unter Unterdruck gehalten, so dass mit dem Öffnen des Ventils und der Betätigung der Saugeinheiten auch der Unterdruck der Vakuumkammer am Eingang der Saugleitung auftritt. Dieser während des Bereitschaftszustands in der Vakuumkammer aufrechterhaltene Unterdruck kann durch eine der Saugeinheiten, vorzugsweise die zweite Saugeinheit bewirkt werden. Die beiden Saugeinheiten und das gegebenfalls vorgesehene Ventil sind daher vorzugsweise individuell mittels einer Steuereinheit steuerbar.

Die Steuereinheit ist vorzugsweise mit Sensoren verbunden, mittels denen der Durchfluss und/oder der Druck in den Anschlussleitungen der Saugeinheiten und/oder der Saugleitung messbar sind.

Die Steuereinheit ist vorzugsweise mit einem Programmodul versehen, mittels dessen die wenigstens eine erste Saugeinheit und die zweite Saugeinheit in Abhängigkeit einer gewählten Saugleistung am Ausgang der Saugleitung und/oder in Abhängigkeit der von den Sensoren abgegebenen Messwerten gesteuert oder geregelt werden. Die erste Saugeinheit weist dazu vorzugsweise eine steuerbare Medienversorgungseinheit auf, die zur Abgabe eines flüssigen oder gasförmigen Treibmediums vorgesehen ist, mittels dessen der erste Unterdruck erzeugt wird.

Die erfindungsgemässe Lösung kann auch nachträglich in bestehende Saugvorrichtungen eingebaut werden, die eine Verdrängerpumpe aufweisen.

Nachfolgend wird die Erfindung anhand von Zeichnungen näher erläutert. Dabei zeigt:
- Fig. 1a: eine erfindungsgemässe Saugvorrichtung mit einer als Strahlpumpe ausgebildeten ersten Saugeinheit 1 und einer als Verdrängerpumpe ausgestalteten zweiten Saugeinheit 2, die über ein Kopplungsstück 121 und eine in einen Behälter oder in eine Vakuumkammer 3 eingesetzte Medientrennvorrichtung 4 mit einer Saugleitung 31 verbunden sind;
- Fig. 1b: die Medientrennvorrichtung 4 von Fig. 1a die einen Teil 342 des Behälters 3 oder einer Behälterabdeckung umfasst;
- Fig. 2: die Saugvorrichtung von Fig. 1a in einer alternativen Ausgestaltung mit einer Vakuumkammer 3 in die eine Medientrennvorrichtung 4 integriert ist;
- Fig. 3: die Saugvorrichtung von Fig. 2 in einer alternativen Ausgestaltung mit einer Steuereinheit 9, mittels der von Sensoren S1, S2, S3, S4 abgegebene Messsignale verarbeitet und die beiden Saugeinheiten 1, 2 sowie ein dem Abschliessen der Vakuumkammer 3 dienendes Ventil 7 und eine Absaugpumpe 8 steuerbar sind;
- Fig. 4: die Saugvorrichtung von Fig. 2 in einer alternativen Ausgestaltung mit zwei mit der Vakuumkammer 3 verbundenen ersten Saugeinheiten 1A, 1B, die seriell hintereinander geschaltet sind und von demselben Treibmedium durchflossen werden; und
- Fig. 5: eine erfindungsgemässe Saugvorrichtung mit einer als Strahlpumpe ausgebildeten ersten Saugeinheit 1 und einer als Gebläsepumpe ausgestalteten zweiten Saugeinheit 2, die in die Vakuumkammer 3 integriert ist.

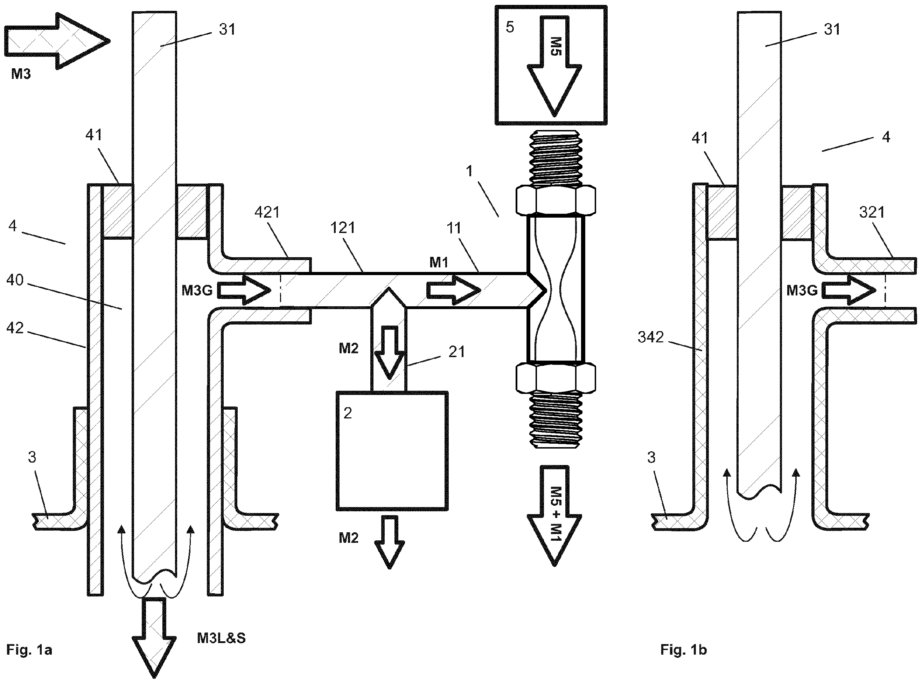

Fig. 1a zeigt eine erfindungsgemässe Saugvorrichtung mit einer als Strahlpumpe ausgebildeten ersten Saugeinheit 1 und einer als Verdrängerpumpe ausgestalteten zweiten Saugeinheit 2, die über ein Kopplungsstück 121 und eine Medientrennvorrichtung 4 mit einer Saugleitung 31 verbunden sind. Das gezeigte Rohrstück kann auch aus festem Material, wie Kunststoff oder Glas, gefertigt und mit einer Saugleitung 31 verbunden sein.

In Fig. 1 ist symbolisch gezeigt, dass die erste Saugeinheit 1 eine Venturi-Düse umfasst, der von einer Medienquelle 5, vorzugsweise einer maschinell betriebenen Luftpumpe, wie einem Verdichter oder Kompressor, das Treibmedium M5 zugeführt wird. Das Treibmedium M5 durchläuft die Venturi-Düse, wonach bei der zentral an die Venturi-Düse angeschlossenen Anschlussleitung 11 ein Unterdruck resultiert. Strahlpumpen anderer Art, wie eine Ejektor-Pumpe, sind ebenfalls einsetzbar.

Die zweite Saugeinheit 2, z.B. eine Membranpumpe oder eine Kolbenpumpe, ist ebenfalls symbolisch gezeigt. Die Anschlussleitung 21 der zweiten Saugeinheit 2 und die Anschlussleitung 11 der ersten Saugeinheit 1 sind zusammengeführt und bilden ein Kopplungsstück 121, welches in einen Anschlussport 421 der Medientrennvorrichtung 4 eingesetzt ist.

Die vorteilhaft aufgebaute Medientrennvorrichtung 4 umfasst ein in einen Behälter 3 eingesetzte Aussenrohr 42, welches mit dem rohrförmigen Anschlussport 421 versehen ist. Innerhalb des Aussenrohrs 42 ist die Saugleitung 31 oder ein mit der Saugleitung 31 verbindbares Innenrohr von einem Abschlusselement 41 gehalten. Das Abschlusselement ist z.B. ein Deckel, der auf das Aussenrohr 42 aufgesetzt oder, wie gezeigt, in dieses eingeschraubt werden kann. Der hohlzylindrische Deckel 41 schliesst an die Innenwand des Aussenrohrs 42 an und umschliesst die Saugleitung oder das Innenrohr 31 dicht abschliessend und koaxial zum Aussenrohr 42 ausgerichtet.

Zwischen der Aussenseite der Saugleitung 31, durch die hindurch das abgesaugte Medium M3 transportiert wird, und der Innenseite des Aussenrohrs 42 ist ein Rückflusskanal 40 mit einem ringförmigen Querschnitt freigehalten. Die Saugleitung 31 ist am Anschlussport 421 vorbeigeführt, so dass ein angesaugter Medienanteil M3L&F, der Flüssigkeit und Feststoffe umfasst, nach unten in den verschlossenen Behälter 3 geführt wird. Lediglich der gasförmige Medienanteil M3G wird durch den Rückflusskanal 40 zurück zum Anschlussport 421 geführt. Der gasförmige Medienanteil M3G gelangt somit über einen nur sehr kurzen Umweg zu den beiden Saugeinheiten 1, 2 weshalb das Medium M3 effizient durch die Saugleitung 31 getrieben werden kann. Das Endstück der Saugleitung 31 kann nicht unmittelbar an den Anschlussport 421 angeschlossen aber doch sehr nahe dran angeordnet werden. Durch den Aufbau eines Unterdrucks innerhalb des Aussenrohrs 42 kann daher rasch ein Medienstrom durch die Saugleitung bewirkt werden. Die Verbindung zwischen dem Anschlussport 421 und der ersten Saugeinheit 1 wird dabei so kurz wie möglich gehalten.

Der gasförmige Medienstrom M3G gelangt durch den Anschlussport 421 in das Kopplungsstück 121 und wird dort in die gasförmigen Medienteilströme M1 und M2 aufgeteilt. Die beiden Saugeinheiten 1, 2 sind in der Lage, innerhalb des Aussenrohrs 42 verzögerungslos einen wirksamen Unterdruck zu erzeugen. Von der zweiten Saugeinheit 2 angesaugtes Material gelangt dabei in den Einflussbereich der ersten Saugeinheit 1 und wird von dieser abtransportiert. Damit die zweite Saugeinheit 2 besonders intensiv auf die erste Saugeinheit 1 einwirken kann, wird die Anschlussleitung 11 der ersten Saugeinheit 1 möglichst kurz gewählt. Der Schnittpunkt der ersten und der zweiten Anschlussleitung 11, 21 wird daher vorzugsweise an den Eingang der ersten Saugeinheit 1 bzw. der Venturi-Düse gelegt. Umgekehrt unterstützt auch die erste Saugeinheit 1 die zweite Saugeinheit 2, indem verzögerungslos ein hoher Unterdruck erzeugt und das Medium M3 effizient angesaugt wird.

Fig. 1b zeigt die Medientrennvorrichtung 4 von Fig. 1a in einer vorzugsweisen Ausgestaltung. Das Aussenrohr 42 der Medientrennvorrichtung 4 von Fig. 1a wird von einem Teil der Vakuumkammer bzw. des Behälters 3 oder der Abdeckung des Behälters 3 gebildet. Diese Lösung kann besonders kostengünstig realisiert werden, da kein separates Teil gefertigt, eingesetzt und abgedichtet werden muss.

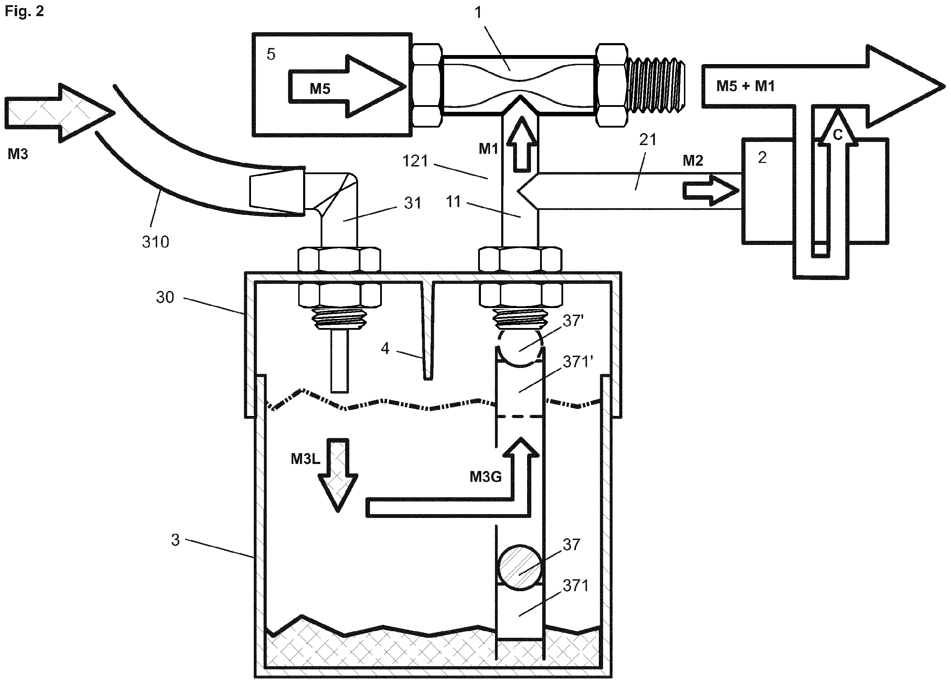

Fig. 2 zeigt die Saugvorrichtung von Fig. 1a in einer alternativen Ausgestaltung mit einer als Sammelbehälter dienenden Vakuumkammer 3 in die eine einfach ausgestaltete Medientrennvorrichtung 4 integriert ist.

Die Saugleitung 31 und das Kopplungsstück 121, welches die Ausgangsleitungen 11, 21 der beiden Saugeinheiten 1, 2 miteinander verbindet, sind mit der Abdeckung 30 des Sammelbehälters bzw. der Vakuumkammer 3 verbunden. Zwischen dem Montagepunkt der Anschlussleitung 31 und dem Montagepunkt des Kopplungsstücks 121 weist die Abdeckung 30 eine Trennwand auf, die als Medientrennvorrichtung 4 dient und verhindert, dass Flüssigkeit und Feststoffe zum Eingang des Kopplungsstücks 121 gelangen können.

Fig. 2 zeigt ferner, dass die Saugleitung 31 ein flexibles Rohr 310 umfasst, welches vom Anwender leicht zu den Absaugpositionen geführt werden kann. Die Saugleitung 31 ist in die Vakuumkammer oder den Flüssigkeitsbehälter 3 eingeführt, aber mit den Anschlussleitungen 11, 21 nicht direkt verbunden. Die beiden Saugeinheiten 1 und 2 erzeugen innerhalb der Vakuumskammer 3 einen Unterdruck und erlauben es, durch die Saugleitung 31 geförderte Teile effizient abzuführen.

Weiter zeigt Fig. 2, dass das Treibmedium vorteilhaft zur Kühlung weiterer Vorrichtungsteile, wie zur Kühlung der zweiten Absaugeinheit 2 verwendet werden kann. Der Kühlstrom C ist symbolisch gezeigt. Auch diesbezüglich resultiert somit eine vorteilhafte Wechselwirkung zwischen den Saugeinheiten.

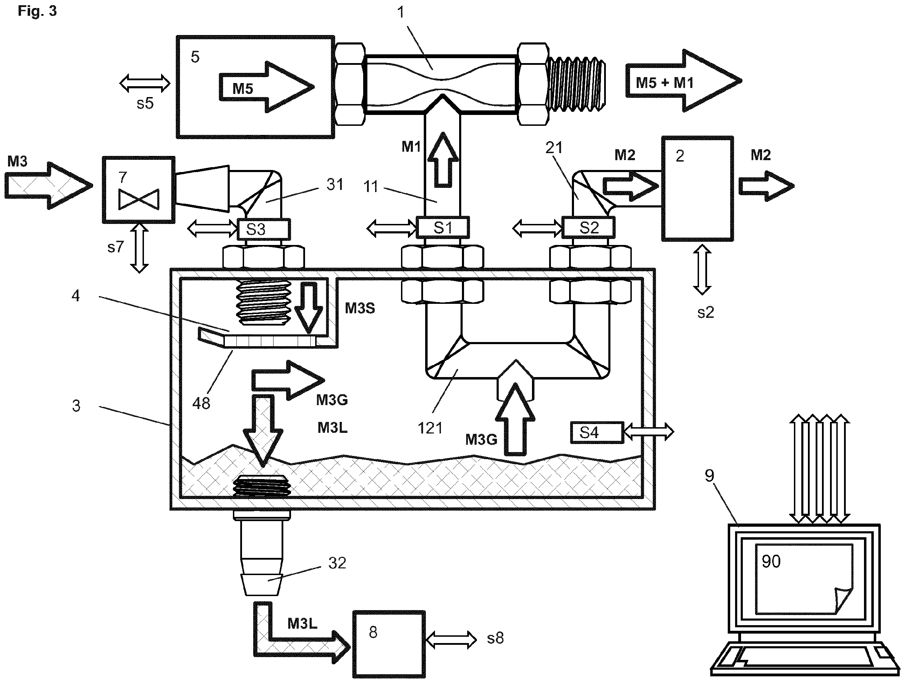

Die erfindungsgemässe Saugvorrichtung kann mittels mechanisch oder elektrisch betriebenen Steuervorrichtungen, Regelvorrichtungen und Verschlussvorrichtungen betrieben und gesichert werden. Wesentlich ist, dass den Saugeinheiten 1, 2 keine Flüssigkeit zugeführt wird. Der Pegelstand der abgesonderten Flüssigkeit M3L wird daher vorzugsweise mechanisch oder elektrisch überwacht. In Fig. 2 ist gezeigt, dass auf einem Schwimmer 371 eine Kugel 37 gelagert ist, die mit steigendem Pegel nach oben gegen die Eintrittsöffnung des Kopplungsstücks 121 geführt wird. Strichpunktiert ist der maximale Flüssigkeitspegel gezeigt, bei dem die vom Schwimmer 371' gehaltene Kugel 37' die Eintrittsöffnung des Kopplungsstücks 121 verschliesst. Mit Erreichen des maximalen Pegelstandes wird vorzugsweise zusätzlich oder alternativ ein Schalter betätigt welcher die Saugeinheiten 1, 2 ausser Betrieb setzt. Bei der Ausgestaltung von Fig. 3 wird hingegen der Pegelstand selbst unter Kontrolle gehalten.

Fig. 3 zeigt die Saugvorrichtung von Fig. 2 in einer alternativen Ausgestaltung mit einer Steuereinheit 9, mittels der von Sensoren S1, S2, S3, S4 abgegebene Messsignale erfasst und die beiden Saugeinheiten 1, 2 sowie ein dem Abschliessen der Vakuumkammer 3 dienendes Ventil 7 und eine Absaugpumpe 8 steuerbar sind.

Die Anschlussleitungen 11 und 21 der beiden Saugeinheiten 1, 2, die innerhalb der Vakuumkammer 3 mit einem Kopplungsstück 121 verbunden sind, sind je mit wenigstens einem Sensor S1, S2 versehen, durch die der in den Anschlussleitungen 11, 21 herrschende Druck und/oder die Durchflussrate der geförderten Medien M1 bzw. M2 messbar sind. Auch in der Saugleitung 31 ist wenigstens ein Sensor S3 vorgesehen, mittels dessen der darin herrschende Druck und/oder die Durchflussrate des angesagten Mediums M3 messbar sind. In der Vakuumkammer 3 ist ein Sensor S4 vorgesehen, welcher den Füllstand der angesaugten Flüssigkeit anzeigt.

An der Unterseite der Vakuumkammer 3 ist ein Anschluss 32 vorgesehen, durch den die Flüssigkeit aus der Vakuumkammer 3 mittels einer Förderpumpe 8 abgesaugt werden kann.

Sofern die Vakuumkammer 3 durch eine Abdeckung 30 abgedeckt ist, wie dies in Fig. 2 gezeigt ist, so wird das Kopplungsstück 121 vorzugsweise innerhalb oder unmittelbar unterhalb der Abdeckung 30 angeordnet.

In die Saugleitung 31 ist ein steuerbares Ventil 7 eingesetzt, mittels der die Vakuumkammer 3 abschliessbar ist, falls keine Flüssigkeit abgesaugt werden soll. Vorzugsweise durch die zweite Saugeinheit 2 kann in der Vakuumkammer 3 somit ein Unterdruck aufrechterhalten werden. Die zweite Saugeinheit 2 wird z.B. zugeschaltet, falls der Druck in der Vakuumkammer 3 einen Schwellwert überschreitet.

Sobald der Anwender eine Flüssigkeit durch die Saugleitung 31 absaugen will, betätigte er einen mit der Steuereinheit 9 verbundenen Schalter, wonach diese über Steuerleitungen s5, s2 und s7 die beiden Saugeinheiten 1, 2 aktiviert und das Ventil 7 öffnet. Die Absaugpumpe 8 wird über die Steuerleitung s8 betätigt.

Die Steuereinheit 9 kann vorzugsweise alle genannten Vorrichtungsteile gemäss einem Steuerprogramm 90 oder entsprechend den von den Sensoren S1, ..., S4 übertragenen Messwerten individuell ansteuern. Vorzugsweise werden mehrere Arbeitspunkte oder Steuerkurven bestimmt, gemäss denen für eine wählbare gesamte Saugleistung die Leistungsanteile der beiden Saugeinheiten 1, 2 individuell festgelegt werden. Die Ansteuerung der beiden Saugeinheiten 1, 2 kann auch in Abhängigkeit des zeitlichen Verlaufs des Betriebs der Saugvorrichtung erfolgen. Bei der Inbetriebnahme der Saugvorrichtung wird das Treibmedium M5 vorzugsweise für kurze Zeit mit erhöhter Intensität durch die erste Saugeinheit 1 geführt, so dass unverzüglich ein starker Druckabfall bzw. Unterdruck in der Saugleitung 31 resultiert und der Medienstrom M3 verzögerungslos in Gang gesetzt wird. Nachdem die Förderwirkung der zweiten Saugeinheit 2 eingetreten ist, kann der Durchfluss des Treibmediums M5 reduziert werden.

Sofern mittels des Sensors S3 Durchflussunterbrüche festgestellt werden, so wird vorzugsweise die erste Saugeinheit 1 auf Impulsbetrieb umgestellt, um die Saugleitung 31 zu reinigen. Vorzugsweise wird ein Impulsbetrieb periodisch durchgeführt, um einer Verstopfung der Leitung vorzubeugen.

Die Medientrennvorrichtung 4 ist bei der Ausgestaltung von Fig. 3 mittels einer gelochten Rückhalteplatte 48 realisiert, die Feststoffe M3S zurückhält, Flüssigkeit M3L ableitet und Luft oder Gas M3G passieren lässt.

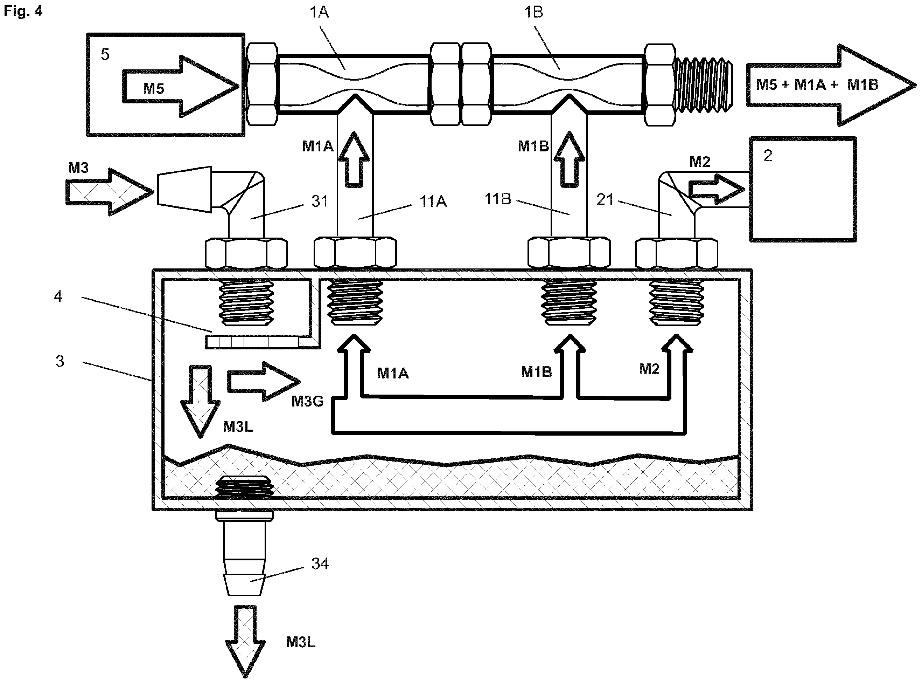

Fig. 4 zeigt die Saugvorrichtung von Fig. 2 in einer alternativen Ausgestaltung mit zwei mit der Vakuumkammer 3 verbundenen ersten Saugeinheiten 1A, 1B, die seriell hintereinander geschaltet sind und von demselben Treibmedium M5 durchflossen werden. Die Saugeinheiten 1A, 1B können hingegen auch parallel zueinander angeordnet und mit der Vakuumkammer 3 verbunden sowie von unterschiedlichen Treibmedien durchlaufen werden. Alternativ können zusätzliche separate erste und/oder zweite Saugeinheiten 1, 2 vorgesehen werden, die vorzugsweise individuell zugeschaltet werden können. Die erfindungsgemässe Saugvorrichtung kann daher erweitert und während des Betriebs an die jeweilige Anwendung angepasst werden. Sofern grössere oder zähflüssigere Flüssigkeitsmengen abgesaugt werden sollen, so können weitere Saugeinheiten 1, 2 zugeschaltet werden. Dies erfolgt manuell oder vorzugsweise automatisch, wenn einer der Sensoren S1, ..., S4 einen ungenügenden Druck anzeigt.

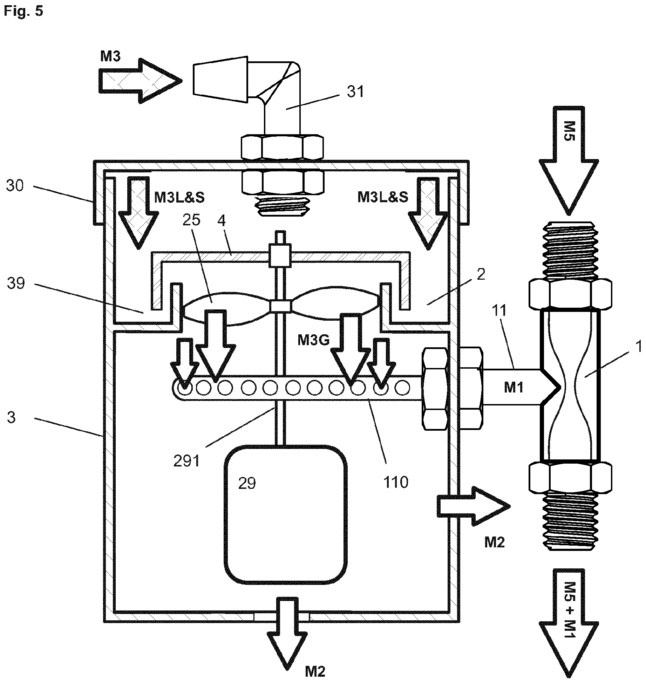

Fig. 5 zeigt eine erfindungsgemässe Saugvorrichtung mit einer als Strahlpumpe ausgebildeten ersten Saugeinheit 1 und einer als Gebläsepumpe ausgestalteten zweiten Saugeinheit 2, die in einen Behälter oder eine Vakuumkammer 3 integriert ist. Die Saugleitung 31 ist mit der Abdeckung 30 der Vakuumkammer 3 verbunden. Die Gebläsepumpe 2 umfasst einen Elektromotor 29 mit einer vertikal ausgerichteten Motorwelle 291, mittels der ein in einem Durchflusskanal gehaltenes Förderwerkzeug, vorliegend ein Propeller 25, drehbar gehalten ist. Der Durchflusskanal wird durch eine umlaufende Rinne 39 gebildet. Oberhalb der Rinne 39 hält die Motorwelle 291 eine als Medientrennvorrichtung 4 dienende Rotorplatte. Auf die Rotorplatte auftreffende Flüssigkeit und Feststoffe M3L&S werden durch Zentrifugalkräfte nach aussen geschleudert und in der Rinne 39 gesammelt. Der gasförmige Medienanteil M3G wird mittels des Propellers 25 hingegen durch den Kanal gefördert.

In dieser vorzugsweisen Ausgestaltung ist die erste Saugeinheit 1 seitlich an der Vakuumkammer 3 befestigt. Ein an die Anschlussleitung 11 anschliessendes Saugrohr 110 ist in die Vakuumkammer 3 hinein geführt. Das mit Öffnungen versehene Saugrohr 110 ist vorzugsweise kreisförmig ausgestaltet und schliesst unterhalb des Propellers 25 endseitig an diesen bzw. an den Rand des Durchflusskanals an. Dadurch wird sichergestellt, dass auch am Rand des Kanals eine intensive Medienförderung und insgesamt ein weitgehend laminarer und somit entsprechend hoher Durchfluss des gasförmigen Mediums M3G resultieren. Weiterhin wird durch die erste Saugeinheit 1 ein Unterdruck innerhalb der Vakuumkammer 3 erzeugt, der durch die Gebläsepumpe 2 allein nicht aufgebaut werden kann. Die Wirksamkeit der zweiten Saugeinheiten bzw. der Gebläsepumpe 2 wird dadurch signifikant erhöht, so dass diese mit geringeren Dimensionen realisiert werden kann. Verschiedene Nachteile der Gebläsepumpe 2, das schwache Vakuum, die grossen Abmessungen und das hohe Gewicht können dadurch deutlich reduziert werden.

Die Gebläsepumpe 2 kann somit auch vorteilhaft mit einer ersten Saugeinheit 1 verbunden werden, die nicht als Strahlpumpe, sondern als Verdrängerpumpe ausgebildet ist. Durch die Verdrängerpumpe 1 kann ein von der Gebläsepumpe 2 allein nicht erreichbares Vakuum aufgebaut werden, während durch die Gebläsepumpe 2 ein hoher Durchfluss des gasförmigen Mediums M3G erzielt wird. In weiteren Ausgestaltungen der Erfindung können daher generell zwei Saugeinheiten 1, 2, wie sie z.B. in den Figuren 1 - 5 gezeigt sind, miteinander kombiniert werden die nach unterschiedlichen physikalischen Prinzipien arbeiten.

Es wurde beschrieben, dass die erfindungsgemässe Saugvorrichtung besonders vorteilhaft für medizinische, insbesondere dental-medizinische Anwendungen einsetzbar ist. Während einem medizinischen Eingriff können störende Flüssigkeiten und Feststoffe verzögerunglos und effizient abgesaugt werden. Dabei kann die Saugleitung kontaktiert sind oder kontaktlos an der Körperoberfläche des Patienten entlang geführt werden, wobei das aufzusaugende Material zuverlässig erfasst und mit einer hohen Durchflussrate abgeführt werden kann. Empfindliche Körperstellen müssen daher nicht zwingend kontaktiert werden, um das Material abzusagen. Auf die Saugleitung können vorteilhaft flüssigkeitsdurchlässige Saugköpfe aufgesetzt werden, die falls überhaupt Körperstellen des Patienten nur schonend kontaktieren und sicherstellen, dass Material abgesaugt werden kann, ohne dass die Saugleitung die Körperoberfläche selbst ansaugt.

Die erfindungsgemässe Lösung ist auch in industriellen Betrieben und beliebigen Werkstätten vorteilhaft einsetzbar. Insbesondere in materialverarbeitenden Betrieben können Metallpartikel, in Betrieben der Chemie und Pharmazie können pulverförmige oder flüssige Chemikalien und in der Nahrungsmittelindustrie können, Flüssigkeiten und Verunreinigungen zuverlässig abgesaugt werden.

Aufgrund des verzögerungsfreien Arbeitens der Saugvorrichtung kann diese besonders vorteilhaft in Produktionsautomaten integriert werden, die mit hohen Taktzyklen arbeiten. Nach einem Arbeitszyklus kann verzögerunglos ein Saugzyklus erfolgen. Die Saugvorrichtung kann dabei vorzugsweise über ein gemeinsames Saugrohr, z.B. durch ein mit dem Kopplungsstück verbundenes Saugrohr in den Arbeitsprozess eingreifen.

## Patentansprüche

1. Vorrichtung zum Absaugen von Flüssigkeit und/oder Feststoffpartikeln insbesondere für medizinische und industrielle Anwendungen mit einer als Strahlpumpe, insbesondere als Venturi-Pumpe oder Ejektor-Pumpe, ausgebildeten ersten Saugeinheit (1), mittels der in einer ersten Anschlussleitung (11) ein erster Unterdruck realisierbar ist, und mit einer als Verdrängerpumpe oder Gebläsepumpe ausgebildeten zweiten Saugeinheit (2), mittels der in einer zweiten Anschlussleitung (21) ein zweiter Unterdruck realisierbar ist, wobei die erste und die zweite Anschlussleitung (11, 21) über eine Vakuumkammer (3) an eine Saugleitung (31) angeschlossen sind, und wobei wenigstens eine Energieversorgungsvorrichtung mit der ersten und der zweiten Saugeinheit (1,2) derart verbindbar oder verbunden ist, dass beide Saugeinheiten (1,2) gemeinsam betrieben werden können, um gemeinsam einen dritten Unterdruck in der Vakuumkammer (3) zu erzeugen.

2. Saugvorrichtung nach Anspruch 1, **dadurch gekennzeichnet, dass** die Saugeinheiten (1,2) über die erste und zweite Anschlussleitung (11,21) und eine Kopplungseinheit (121) an die Saugleitung (31) angeschlossen sind, sodass ein durch die Saugleitung (31) angesaugter gasförmiger Medienstrom in zwei Teilströme aufteilt wird, die den Saugeinheiten (1,2) getrennt voneinander zugeführt werden.

3. Saugvorrichtung nach Anspruch 1 oder 2, **dadurch gekennzeichnet, dass** die Saugleitung (31) über eine Medientrennvorrichtung (4) mit den beiden Saugeinheiten (1,2) verbunden ist.

4. Saugvorrichtung nach Anspruch 3, **dadurch gekennzeichnet, dass** die Medientrennvorrichtung (4) ein in die Vakuumkammer (3) eingesetztes Aussenrohr (42) mit wenigstens einem Anschlussport (421) aufweist, an den die Saugeinheiten (1,2) einzeln oder über eine Kopplungsvorrichtung (121) angeschlossen sind, wobei die Saugleitung (31) oder ein daran vorgesehenes Verlängerungsstück das Aussenrohr (42) vorzugsweise koaxial durchläuft und am Anschlussport (421) vorbei geführt ist.

5. Saugvorrichtung nach einem der Ansprüche 1 - 4, **dadurch gekennzeichnet, dass** die Anschlussleitungen (11,21) der Saugeinheiten (1,2) innerhalb oder ausserhalb der Vakuumkammer (3) mit der Kopplungseinheit verbunden ist, die durch ein Verbindungsteil (121) oder durch die Vakuumkammer (3) gebildet wird.

6. Saugvorrichtung nach Anspruch 5, **dadurch gekennzeichnet, dass** die erste Saugeinheit (1), die zweite Saugeinheit (2) und/oder die Saugleitung (31) an eine Abdeckung (30) angeschlossen sind, mittels der die Vakuumkammer (3) abschliessbar ist.

7. Saugvorrichtung nach einem der Ansprüche 1 - 6, **dadurch gekennzeichnet, dass** zwei oder mehr erste Saugeinheiten (1A,1B) seriell oder parallel zueinander angeordnet mit der Kopplungseinheit (121) oder der Vakuumeinheit (3) verbunden sind.

8. Saugvorrichtung nach einem der Ansprüche 1 - 7, **dadurch gekennzeichnet, dass** die Saugleitung (31) über ein steuerbares Ventil (7) mit der Vakuumkammer (3) verbunden ist, welches für den Absaugvorgang geöffnet werden kann.

9. Saugvorrichtung nach einem der Ansprüche 1 - 8, **dadurch gekennzeichnet, dass** die zweite Saugeinheit (2) eine Kolbenpumpe, eine Membranpumpe oder eine Gebläsepumpe, aber keine peristaltische Pumpe ist.

10. Saugvorrichtung nach Anspruch 9, **dadurch gekennzeichnet, dass** die als Gebläsepumpe ausgebildete und mit einem Gebläsemotor (29) und wenigstens einem Gebläserotor (25) versehene zweite Saugeinheit (2) in die Vakuumkammer (3) integriert ist, an die die erste Saugeinheit (1) angeschlossen ist.

11. Saugvorrichtung nach Anspruch 9 oder 10, **dadurch gekennzeichnet, dass** die erste Saugeinheit (1) an ein mit Öffnungen versehenes, vorzugsweise ringförmig verlaufendes Saugrohr (110) angeschlossen ist, welches vorzugsweise im peripheren Bereich des Gebläserotors (25) verläuft.

12. Saugvorrichtung nach Anspruch 9, 10 oder 11, **dadurch gekennzeichnet, dass** der Gebläsemotor (29) einen Spezialrotor (4) trägt, der als Zentrifugalabscheider dient.

13. Saugvorrichtung nach einem der Ansprüche 1 -12, **dadurch gekennzeichnet, dass** eine mit der ersten und zweiten Saugeinheit (1,2) verbundene Steuereinheit (9) vorgesehen ist, die vorzugsweise mit Sensoren (S1, S2, S3) verbunden ist, mittels denen der Durchfluss und/oder der Druck in den Anschlussleitungen (11,21) der Saugeinheiten (1,2), und/oder der Saugleitung (31) messbar ist.

14. Saugvorrichtung nach Anspruch 13, **dadurch gekennzeichnet, dass** die Steuereinheit (9) mit einem Programmodul (90) versehen ist, mittels dessen die wenigstens eine erste Saugeinheit (1) und die zweite Saugeinheit (2) in Abhängigkeit einer gewünschten Saugleistung am Ausgang der Saugleitung (31) und/oder in Abhängigkeit der von den Sensoren (S1, S2, S3) abgegebenen Messwerten steuerbar oder regelbar sind.

15. Saugvorrichtung nach einem der Ansprüche 1 - 14, **dadurch gekennzeichnet, dass** die erste Saugeinheit (1) eine vorzugsweise steuerbare Medienversorgungseinheit (5) umfasst, die zur Abgabe eines flüssigen oder gasförmigen Treibmediums vorgesehen ist, mittels dessen der erste Unterdruck erzeugt wird.

## Claims

1. Device for aspirating a liquid and/or solid particles particularly for medical or industrial applications with a first aspiration unit (1) designed as a jet pump, particularly a venturi pump or an ejector pump, with which in a first connecting line (11) a first negative pressure can be realised, and with a second aspiration unit (2) designed as a positive displacement pump or a blower pump, with which in a second connecting line (21) a second negative pressure can be realised, wherein the first and the second connecting line (11, 21) are connected via a vacuum chamber (3) to an aspiration line (31), and wherein at least one energy supply device is connected or connectable to the first and the second aspiration unit (1,2) such, that both aspiration units (1,2) can be operated in common, in order to generate in common a third negative pressure in the vacuum chamber (3).

2. Aspiration device according to claim 1, **characterised in that** the aspiration units (1,2) are connected via the first and second connecting line (11,21) and a coupling unit (121) to the aspiration line (31), so that a gaseous media stream aspirated through the aspiration line (31) is divided into two partial streams that are guided separated from one another to the aspiration units (1,2).

3. Aspiration device according to claim 1 or 2, **characterised in that** the aspiration line (31) is connected via a media separation device (4) to the two aspiration units (1,2).

4. Aspiration device according to claim 3, **characterised in that** the media separation device (4) is provided with an outer tube (42) that is inserted into the vacuum chamber (3) and that is provided with at least one connecting port (421), to which the aspiration units (1,2) are connected individually or via a coupling device (121), wherein the aspiration line (31) or an extension member provided thereon preferably coaxially traverses the outer tube (42) and is guided past the connecting port (421).

5. Aspiration device according to one of the claims 1 - 4, **characterised in that** the connecting lines (11,21) of the aspiration units (1,2) are connected inside or outside of the vacuum chamber (3) to the coupling unit, which is formed by a connecting member (121) or by the vacuum chamber (3).

6. Aspiration device according to claim 5, **characterised in that** the first aspiration unit (1), the second aspiration unit (2) and/or the aspiration line (31) are mounted on a cover (30), with which the vacuum chamber (3) can be closed.

7. Aspiration device according to one of the claims 1 - 6, **characterised in that** two or more first aspiration units (1A,1B) are connected, arranged in series or in parallel to one another, to the coupling unit (121) or the vacuum unit (3).

8. Aspiration device according to one of the claims 1 - 7, **characterised in that** the aspiration line (31) is connected via a controllable valve (7), which can be opened for the aspiration process, to the vacuum chamber (3).

9. Aspiration device according to one of the claims 1 - 8, **characterised in that** the second aspiration unit (2) is a piston pump, a diaphragm pump or a blower pump, but not a peristaltic pump.

10. Aspiration device according to claim 9, **characterised in that** the second aspiration unit (2), which is designed as a blower pump and is provided with a blower motor (29) and at least one blower rotor (25), is integrated into the vacuum chamber (3) to which the first aspiration unit (1) is connected.

11. Aspiration device according to claim 9 or 10, **characterised in that** the first aspiration unit (1) is connected to a preferably annular aspiration tube (110) that is provided with openings and that preferably extends in the peripheral area of the blower rotor (25) .

12. Aspiration device according to claim 9, 10 or 11, **characterised in that** the blower motor (29) supports a special rotor (4), which serves as a centrifugal separator.

13. Aspiration device according to one of the claims 1 -12, **characterised in that** a control unit (9) is provided, which is connected to the first and second aspiration unit (1,2) and which is preferably connected to sensors (S1, S2, S3) with which the flow and/or the pressure in den connecting lines (11,21) of the aspiration units (1,2) and/or the aspiration line (31) is measurable.

14. Aspiration device according to claim 13, **characterised in that** the control unit (9) is provided with a program module (90), with which the at least one first aspiration unit (1) and the second aspiration unit (2) are controllable or adjustable depending on a desired throughput at the exit of the aspiration line (31) and/or depending on the measurands delivered by the sensors (S1, S2, S3).

15. Aspiration device according to one of the claims 1 - 14, **characterised in that** the first aspiration unit (1) comprises a preferably controllable media supply unit (5), which is provided for delivering a liquid or gaseous propellant, by means of which the first negative pressure is generated.

## Revendications

1. Dispositif pour aspirer des liquides et/ou des particules solides, en particulier pour des applications médicales et industrielles, avec une première unité d'aspiration (1) conçue comme pompe à jet, en particulier pompe venturi ou éjecteur, avec laquelle une première dépression peut être réalisée dans un premier conduit de raccordement (11), et avec une deuxième unité d'aspiration (2) conçu comme pompe à déplacement ou pompe à ventilateur, avec laquelle une deuxième dépression peut être réalisée dans un deuxième conduit de raccordement (21), le premier et le deuxième conduit de raccordement (11, 21) étant connectés par une chambre à vide (3) à un conduit d'aspiration (31), et au moins un dispositif d'alimentation électrique étant connecté ou pouvant être connecté à la première et la deuxième unité d'aspiration (1, 2) de manière à ce que les deux unités d'aspiration (1, 2) pouvant être opérées ensemble pour créer ensemble une troisième dépression dans la chambre à vide (3).

2. Dispositif d'aspiration selon la revendication 1, **caractérisé en ce que** les deux unités d'aspiration (1,2) sont connectées par le premier et le deuxième conduit de raccordement (11, 21) et une unité d'accouplement (121) au conduit d'aspiration (31), tel qu'un flux de médias gazeux aspiré par le conduit d'aspiration (31) est divisé dans deux flux partiels qui sont amenés vers les unités d'aspiration (1, 2) séparés l'un de l'autre.

3. Dispositif d'aspiration selon la revendication 1 ou 2, **caractérisé en ce que** le conduit d'aspiration (31) est connecté par un dispositif de séparation de médias (4) aux deux unités d'aspiration (1,2).

4. Dispositif d'aspiration selon la revendication 3, **caractérisé en ce que** le dispositif de séparation de médias (4) présente un tuyaux extérieur (42) inséré dans la chambre à vide (3), ledit tuyaux extérieur (42) présentant au moins un point de connexion (421), auquel les unités d'aspiration (1, 2) sont connectées séparément ou par une unité d'accouplement (121), le conduit d'aspiration (31) ou une pièce d'extension prévu là-dessus traversant le tuyaux extérieur (42) de préférence de manière coaxiale et étant guidé autour du point de connexion (421).

5. Dispositif d'aspiration selon une des revendications 1 - 4, **caractérisé en ce que** les conduits de raccordement (11,21) des unités d'aspiration (1,2) sont connectés à l'intérieur ou à l'extérieur de la chambre à vide (3) à l'unité d'accouplement, qui est formé d'une pièce de raccordement (121) ou la chambre à vide (3).

6. Dispositif d'aspiration selon la revendication 5, **caractérisé en ce que** la première unité d'aspiration (1), la deuxième unité d'aspiration (2) et/ou le conduit d'aspiration (31) sont connecté à un couvercle (30), par lequel la chambre à vide (3) peut être fermée.

7. Dispositif d'aspiration selon une des revendications 1 - 6, **caractérisé en ce que** deux ou plusieurs premières unité d'aspiration (1A,1B) sont connectées en série ou en parallèle avec l'unité d'accouplement (121) ou la chambre à vide (3).

8. Dispositif d'aspiration selon une des revendications 1 - 7, **caractérisé en ce que** le conduit d'aspiration (31) est connecté à la chambre à vide (3) par une vanne réglable (7), qui peut être ouvert pour le processus d'aspiration.

9. Dispositif d'aspiration selon une des revendications 1 - 8, **caractérisé en ce que** la deuxième unité d'aspiration (2) est une pompe à piston, une pompe à diaphragme, ou une pompe à ventilateur, mais pas une pompe péristaltique.

10. Dispositif d'aspiration selon la revendication 9, **caractérisé en ce que** la deuxième unité d'aspiration (2) conçu comme pompe à ventilateur et muni d'un moteur de ventilateur (29) et au moins un rotor de ventilateur (25) est intégrée dans la chambre à vide (3) et connecté à la première unité d'aspiration (1).

11. Dispositif d'aspiration selon la revendication 9 ou 10, **caractérisé en ce que** la première unité d'aspiration (1) est connecté à un tuyau d'aspiration (110) présentant des ouvertures et de préférence conçu en forme d'anneau, ledit tuyaux d'aspiration (110) s'étendant de préférence dans la zone périptère du rotor de ventilateur (25).

12. Dispositif d'aspiration selon la revendication 9, 10 ou 11, **caractérisé en ce que** le moteur de ventilateur (29) porte un rotor spécial (4), qui sert comme séparateur centrifuge.

13. Dispositif d'aspiration selon une des revendications 1 -12, **caractérisé en ce qu'**une unité de pilotage (9) connecté à la première et deuxième unité d'aspiration (1, 2) est prévue, qui est de préférence connecté à des capteurs (S1, S2, S3), par lesquels le flux et/ou la pression dans les conduit de raccordement (11, 21) des unités d'aspiration (1, 2) et/ou du conduit d'aspiration (31) peuvent être déterminés.

14. Dispositif d'aspiration selon la revendication 13, **caractérisé en ce que** l'unité de pilotage (9) est muni d'un module de programmation (90), par lequel la au moins une première unité d'aspiration (1) et la deuxième unité d'aspiration (2) sont dirigeable et réglable en fonction de la puissance d'aspiration souhaité à la sortie du conduit d'aspiration (31) et/ou en fonction des valeurs mesurées par les capteurs (S1, S2, S3).

15. Dispositif d'aspiration selon une des revendications 1 - 14, **caractérisé en ce que** la première unité d'aspiration (1) comprend une unité d'alimentation de médias (5) de préférence dirigeable, qui est destiné à fournir un média d'entraînement liquide ou gazeux, avec lequel la première dépression est réalisé.
